# DEMANDE DE BREVET EUROPEEN

(11) **EP 1 649 898 A2**
(43) Date de publication de la demande: **26.04.2006**
(21) Numéro de dépôt: 05292158.2
(22) Date de dépôt: 13.10.2005
(51) Int. Cl.: A61Q 5/06, A61K 8/40

(54) **Composition de coloration comprenant un pigment et au moins un monomère électrophile spécifique**

(30) Priorité: 13.10.2004 FR 0410800
(71) Demandeur: L'OREAL, 75008 Paris (FR)
(72) Inventeur: Brun, Gaëlle, 75015 Paris (FR)
(74) Mandataire: Fevrier, Murielle Françoise E.

(57) **Abrégé**

La présente invention a pour objet un procédé de coloration des fibres kératiniques comprenant l'application sur les matières kératiniques d'une composition de coloration comprenant, dans un milieu de coloration approprié, au moins un pigment et au moins un monomère cyanoacrylate de formule (1) 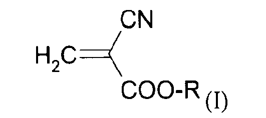 dans laquelle R représente un radical alkyle ou alcoxyalkyle.

La composition conforme à la présente invention permet d'obtenir une coloration visible sur une matière kératinique foncée

## Description

La présente invention a pour objet une composition de coloration des matières kératiniques, en particulier des fibres kératiniques telles que les cheveux, comprenant au moins un pigment et au moins un monomère électrophile particulier ainsi qu'un procédé de coloration des matières kératiniques à partir de cette composition.

Dans le domaine de la coloration des fibres kératiniques, il est déjà connu de colorer des fibres kératiniques par différentes techniques à partir de colorants directs ou de pigments pour des colorations non permanente ou de précurseurs de colorant pour des colorations permanentes.

La coloration non permanente ou coloration directe consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Certains de ces colorants peuvent être utilisés dans des conditions éclaircissantes ce qui permet d'obtenir des colorations visibles sur des cheveux foncés.

Il est aussi connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs et les colorations qui en résultent sont permanentes, puissantes, et résistantes aux agents extérieurs, notamment à la lumière, aux intempéries, aux lavages, à la transpiration et aux frottements.

Pour être visible sur cheveux foncés, ces deux techniques de coloration nécessitent une décoloration préalable ou simultanée des fibres kératiniques. Cette étape de décoloration mise en oeuvre avec un agent oxydant tel que le peroxyde d'hydrogène ou de persels entraîne une dégradation non négligeable des fibres kératiniques ce qui altère leurs propriétés cosmétiques. Les cheveux ont alors tendance à devenir rêches, plus difficilement démêlables et plus fragiles.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR 2 741 530, qui préconise l'utilisation pour la coloration des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient d'avoir une faible résistance aux shampooings.

Par ailleurs, il est connu de la demande de brevet FR 2 833 489 des compositions de traitement des cheveux à partir de compositions comprenant des monomères électrophiles. Une telle composition permet d'obtenir des cheveux parfaitement gainés et non gras.

Le but de la présente invention est de fournir de nouvelles compositions pour la coloration des matières kératiniques, et en particulier des fibres kératiniques telles que les cheveux, qui permettent d'obtenir des colorations visibles sur cheveux foncés sans qu'il soit nécessaire d'éclaircir ou de décolorer les fibres et qui présentent une bonne résistance aux shampooings.

Ce but est atteint avec la présente invention qui a pour objet un procédé de coloration des matières kératiniques, en particulier les fibres kératiniques tels que les cheveux, à partir d'une composition comprenant, dans un milieu de coloration approprié, au moins un pigment et au moins un monomère cyanoacrylate de formule (I) 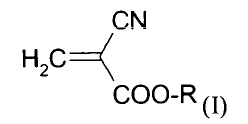 dans laquelle R représente un radical alkyle ou alcoxyalkyle.

L'invention a aussi pour objet une composition de coloration des matières kératiniques comprenant, dans un milieu de coloration approprié, au moins un pigment en quantité supérieure à 5 % en poids du poids total de la composition et au moins un monomère cyanoacrylate de formule (I)

Le procédé conforme à la présente invention permet d'obtenir une coloration visible sur une matière kératinique foncée. En particulier, dans le cas des fibres kératiniques foncées, on obtient une coloration très visible sans qu'il soit nécessaire d'éclaircir ou de décolorer les fibres kératiniques et par conséquent sans dégradation physique des fibres kératiniques.

De plus, cette coloration présente une bonne résistance aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis à vis des shampooings.

Compte tenu de la variété de pigments qui peuvent être utilisée dans la composition de l'invention, il est possible d'obtenir des colorations dans des nuances variées puissantes et esthétiques et qui peuvent être chromatiques.

Un autre objet de l'invention concerne un kit comprenant un pigment et un monomère cyanoacrylate de formule (I).

Dans le cadre de l'invention, on entend par pigment toute entité organique et / ou minérale dont la solubilité dans l'eau est inférieure à 0,01 % à 20 °C, de préférence inférieure à 0,0001 %, et présentant une absorption entre 350 et 700 nm, de préférence une absorption avec un maximum.

Les pigments utiles dans la présente invention sont choisis parmi tous les pigments organiques et / ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : Ta₂O₅, Ti₃O₅, Ti₂O₃, TiO, ZrO₂ en mélange avec TiO₂, ZrO₂, Nb₂O₅, CeO₂, ZnS.

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références Cl 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2679771.

On peut utiliser des pâtes pigmentaires de pigment organique telles que les produits vendus par la société HOECHST sous le nom :
- JAUNE COSMENYL IOG : Pigment YELLOW 3 (Cl 11710) ;
- JAUNE COSMENYL G : Pigment YELLOW 1 (Cl 11680) ;
- ORANGE COSMENYL GR : Pigment ORANGE 43 (Cl 71105) ;
- ROUGE COSMENYL R : Pigment RED 4 (Cl 12085) ;
- CARMIN COSMENYL FB : Pigment RED 5 (Cl 12490) ;
- VIOLET COSMENYL RL : Pigment VIOLET 23 (Cl 51319) ;
- BLEU COSMENYL A2R : Pigment BLUE 15.1 (Cl 74160) ;
- VERT COSMENYL GG : Pigment GREEN 7 (Cl 74260) ;
- NOIR COSMENYL R : Pigment BLACK 7 (Cl 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45430),D&CRed7(CI 15 850:1),D & C Red 4 (CI 155 10), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Colorona commercialisée par Merck (Mica-TiO2-Fe2O3).

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré des pigments autres que les pigments blancs.

La taille du pigment utile dans le cadre de la présente invention est généralement comprise entre 10 nm et 200 µm, de préférence entre 20 nm et 80 µm, et plus préférentiellement entre 30 nm et 50 µm.

Le ou les pigments sont chacun généralement présents dans la composition conforme à l'invention dans des quantités généralement comprises entre 0,05 et 50 % du poids total de la composition, de préférence de 0,1 à 35 %.

Dans le cadre de l'invention, les radicaux alkyle ou alcoxyalkyle peut être linéaires, ramifiés pouvant être cycliques.

A titre de monomères cyanoacrylates de formule (I), on peut citer les monomères électrophiles le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Selon un mode de réalisation particulier, les monomères cyanoacrylates de formule (I) sont choisis parmi ceux comprenant des radicaux R alkyle ou alcoxyalkyle de 1 à 10 atomes de carbone, de préférence de 6 à 10 atomes de carbones.

Selon une varinte, le monomère cyanoacrylate est choisi parmi les alkyl(C6-C 10)-cyanoacrylates. Selon un mode de réalisation préféré, le monomère cyanoacrylate est l'octyl 2-cyanoacrylate, linéaire ou ramifié.

Les monomères le plus particulièrement préférés sont ceux de formule suivante
et leurs mélanges : 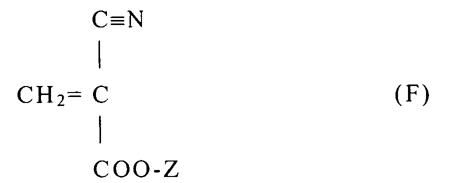 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés dans la composition de l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymère, oligomère ou dendritique peut être statistique, en position terminale ou sous forme de blocs.

Dans la composition de l'invention, la quantité de monomères cyanoacrylate est comprise entre 0,1 et 80 % en poids du poids total de la composition, de préférence entre 1 et 50 %.

Dans le cadre de l'invention, les monomères électrophiles cyanoacrylates de formule (I) sont des monomères capables de polymériser par voie anionique en présence d'un agent nucléophile. Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par " carbanion ", les espèces chimiques définies dans " Advanced Organic Chemistry, Third Edition ", de Jerry March, page 141.

Les agents nucléophiles peuvent être peut être appliqués indépendamment de la composition de l'invention. Il peut aussi être ajouté à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C^{≡}C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, Ph représentant un groupe phényle ; Ar représentant un groupe aryle et R représentant un groupe alkyle en C1-C10.

Les monomères cyanoacrylates de formule (I) selon la présente invention peut être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

Le milieu de coloration approprié utilisé dans la composition de l'invention est de préférence un milieu anhydre et non hygroscopique. On entend par " milieu anhydre ", un milieu contenant moins de 1 % d'eau.

Selon ce mode de réalisation, le milieu de coloration de la composition de l'invention est de préférence choisi parmi :
- les alcools aromatiques tels que l'alcool benzylique ;
- les alcools gras ;
- les polyols modifiés ou non tels que le glycérol, le glycol, le propylène glycol, le dipropylène glycol, le butylène glycol, le butyle diglycol ;
- les silicones volatiles ou non telles que la cylopentasiloxane, la cyclohexasiloxane, les polydiméthylsiloxanes modifiées ou non par des fonctions phényl et/ou siloxy et/ou silanol et/ou amine et/ou imine et/ou fluoroalkyl et/ou carboxylique et/ou betaïne et/ou ammonium quaternaire et/ou etc. ;
- les huiles minérales, organiques ou végétales ;
- les cires oxyéthylénées ou non, les paraffines, les alcanes et plus particulièrement les alcanes de C₅ à C₁₀;
- les acides gras, les amides grasses, les esters gras et plus particulièrement les benzoates ou les salicylates d'alcool gras.

Selon un mode de réalisation préféré, le milieu est constitué par une silicone tels que les polydiméthylsiloxanes et les polydiméthylsiloxanes modifiées.

Le milieu de coloration de la composition de l'invention peut aussi se présenter sous forme d'une émulsion et/ou être encapsulé, les monomères électrophiles étant maintenus dans un milieu anhydre jusqu'au moment de l'utilisation. Lorsque le milieu de coloration est une émulsion, cette émulsion est par exemple constituée par une phase dispersée ou continue qui peut être constituée par de l'eau, des alcools aliphatiques en C1-C4 ou leurs mélanges et une phase organique anhydre comprenant le monomère.Dans le cas des capsules ou microcapsules, la capsule peut contenir le monomères dans un milieu anhydre et être dispersées dans un milieu anhydre tel que défini précédemment, de l'eau, des alcools aliphatiques en C1-C4 ou leurs mélanges.

De préférence, les composés organiques sont choisis parmi les composés liquides à la température de 25°C et sous 105 Pa (760mm de Hg).

On peut également introduire dans la composition de l'invention des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, les acides organiques tels qu'un acide sulfonique ou l'acide phosphorique, l'acide acétique, la lactone, le trifluorure de bore, l'hydroquinone et ses dérivés tels que l'hydroquinone monéthyléther, la tertiobutylhydroquinone, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, et leurs mélanges. Les groupements alkyle désignent de préférence des groupements ayant 1 à 6 atomes de carbone.

La concentration en inhibiteur dans la composition de l'invention peut être comprise entre 10 ppm et 10 %, et plus préférentiellement entre 50 ppm et 5% en poids.

La composition de l'invention peut aussi contenir un ou plusieurs polymères ne présentant pas de réactivité sur les monomères cyanoacrylates et capable d'augmenter la viscosité de la composition. L'augmentation de la viscosité permet de réduire la vitesse de polymérisation des monomères de cyanoacrylate. Pour ce faire, on peut ajouter à la composition de l'invention et de façon non exhaustive le polyméthylméthacrylate (PMMA) ou encore les copolymères à base de cyanoacrylate tels qu'ils sont décrits dans le brevet US 6,224,622.

La composition de l'invention peut aussi contenir des charges. Cela comprend à titre non limitatif des particules incolores ou blanches, minérales ou de synthèse, lamellaires ou non lamellaires. Elles peuvent être présentes à raison de 0 à 48 % en poids, par rapport au poids total de la composition, de préférence 0,01 à 30 % en poids, et mieux de 0,02 % à 20 % en poids. On peut notamment citer le talc, le stéarate de zinc, le mica, le kaolin, les poudres de polyamide (Nylon@) (Orgasol de chez Atochem), les poudres de polyéthylène, les poudres de polymères de tétrafluoroéthylène (TéflonB), l'amidon, le nitrure de bore, des microsphères polymériques telles que celles de chlorure de polyvinylidène/acrylonitrile comme l'Expancel (Nobel Industrie), de copolymères d'acide acrylique (PolytrapB de la société Dow Corning) et les microbilles de résine de silicone (TospearlsO de Toshiba, par exemple), les organopolysiloxanes élastomères.

La composition peut aussi contenir les actifs cosmétiques habituellement utilisés. On peut citer à titre non limitatif les agents réducteurs, les agents oxydants, les corps gras, les silicones, les agents épaississants, les adoucissants, les agents anti-mousse, les agents hydratants, les agents émollients, les agents alcalinisants, les élastomères, les plastifiants, les filtres solaires, les argiles, les minéraux colloïdaux, les parfums, les peptisants, les conservateurs, les tensioactifs anioniques cationiques, amphotères, zwitterionqiues ou non-ionques, les polymères fixants ou non, les polymères conditionneurs, les protéines, les vitamines...

La composition peut aussi contenir des particules ou poudres métalliques telles que des particules ou poudres d'aluminium, de cuivre, etc.

Ces compositions peuvent se présenter sous des formes diverses, telles que des lotions, des sprays, des mousses et être appliquées sous forme de shampooing ou d'après-shampooing.

Dans le cas des sprays, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Selon le procédé de l'invention, la composition de l'invention est appliquée sur les matières kératiniques, en particulier les fibres kératiniques telles que les cheveux, en présence d'un agent nucléophile.

Selon un mode de réalisation particulier du procédé de l'invention, l'agent nucléophile susceptible d'initier la polymérisation du monomère cyanoacrylate peut être appliqué au préalable sur les fibres kératiniques. L'agent nucléophile peut être utilisé pur, en solution, sous forme d'une émulsion ou être encapsulé. Il peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Selon une variante, le procédé de l'invention peut être mise en oeuvre en plusieurs étapes : une première étape qui consiste à appliquer une composition contenant le ou les pigments sur les fibres et une seconde étape qui consiste à appliquer une composition contenant le monomère cyanoacrylate de formule (I), l'agent nucléophile étant présent dans la composition contenant le pigment ou dans une composition séparée.

Selon cette variante, la composition contenant le ou les pigments est de préférence une solution aqueuse de pigments ce qui permet une humidification de la fibre et l'initiation de la polymérisation lorsque le monomère cyanoacrylate est appliqué.

Selon le procédé de l'invention, un mode de réalisation préféré consiste à appliquer soit le monomère électrophile et les pigments à partir d'une même composition soit d'appliquer dans un premier temps le pigment, puis le monomère électrophile.

Le procédé de l'invention peut comprendre des étapes additionnels intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

Afin d'améliorer entre autre l'adhésion du poly(cyanoacrylate) formé in situ, la fibre peut être prétraitée avec tous types de polymères.

Pour moduler la cinétique de polymérisation anionique, on peut également augmenter la nucléophilie de la fibre par transformation chimique des fibres kératiniques. A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants: thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formo-sulfoxylate de zinc, thioglycolate d'isooctyle, dl-cystéine, thioglycolate de monoéthanolamine.

L'application de la composition de l'invention peut aussi être précédée d'un traitement capillaire comme une coloration directe ou d'oxydation.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

L'invention a aussi pour objet un kit de coloration comprenant une première composition qui contient un pigment et une seconde composition qui contient le ou les monomères cyanoacrylates de formule (I) et éventuellement une troisième composition qui contient l'agent nucléophile. Selon ce mode de réalisation, la composition contenant le ou les pigments est une composition aqueuse et la composition contenant le ou les monomères est une composition anhydre.

Selon une première variante, le kit comprend une première composition anhydre qui comprend le ou les pigments et le ou les monomères cyanoacrylates et une deuxième composition qui comprend un agent nucléophile.

Les exemples suivants non limitatifs permettent d'illustrer l'invention sans en limiter sa portée.

### EXEMPLES

Des essais ont été réalisés en utilisant les composés suivants :
Monomère : 2-octyl-2-cyanoacrylate stabilisé avec 1 % d'acide phosphorique connu sous le nom de RITE LOK CON895, commercialisé par la Société CHEMENCE
Pigment 1: Oxyde de fer brun CI 77491 commercialisé par LCW,
Pigment 2 : Nacre mica-oxyde de titane à reflets interférentiels dorés commercialisée par Eckart sous le nom Prestige Gold.
Milieu de coloration :
   50% mélange poly dimethylsiloxane alpha-omega dihydroxyle /cyclopenta dimethylsiloxane (14.7/85.3) commercialisé par Dow Corning sous le nom DC 1501 Fluid
   50% de cyclopentadimethylsiloxane commercialisé par Dow Corning sous le nom DC 245 Fluid

### Exemple 1 :

Une composition aqueuse est préparée avec 10% de pigment 1. 0,5g de cette solution aqueuse est appliqué sur 1g d'une mèche de cheveux naturels propres et secs de hauteur de ton égale à 4 ce qui correspond à une nuance naturelle châtain selon la classification des nuances naturelles décrite dans "Science des Traitements Capillaires" de C. ZVIAK, Ed. Masson 1988, p. 278.

La mèche est ensuite séchée au casque, puis humidifiée avec 0,5g d'eau. Sur cette mèche humidifiée, on applique 0,5g d'une composition contenant le milieu de coloration décrit ci dessus et 10% en poids de monomères cyanoacrylates.

Après 15 minutes de pose la mèche est séchée pendant 2 minutes au sèche-cheveux.

La mèche obtenue est colorée en orangé et la couleur obtenue est rémanente à au moins six shampooings.

### Exemple 2

On prépare une composition contenant 10 % de pigment 2 dans le milieu de coloration décrit ci dessus. Le monomère cyanoacrylate est ajouté à cette composition de manière à obtenir une concentration finale de 10% en poids de monomères. 0,5g de cette composition est appliqué sur une mèche de cheveux naturels propres et secs de hauteur de ton égale à 4, humidifiée avec 0,5g d'eau.

Après 15 minutes de pose, la mèche est séchée pendant 2 minutes au sèche-cheveux. On obtient une mèche colorée en doré

La coloration ainsi obtenue est très résistante aux shampooings.

### Exemple 3. Monomère methylheptylcyanoacrylate

La composition suivante est réalisée :

| | |
|---|---|
| DC 1501 Fluid | 40g |
| DC 245 Fluid | 40g |
| Nacre mica -oxyde de fer brun Prestige bronze Eckart | 10g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.
La mèche est colorée et la coloration obtenue est résistante au shampooing.

### Exemple 4. Monomère methylheptylcyanoacrylate avec acide acétique

La composition suivante **A** est réalisée :

| | |
|---|---|
| méthylheptylcyanoacrylate de Chemence | 97.5g |
| acide acétique glacial | 2.5g |

La composition suivante **B** est réalisée :

| | |
|---|---|
| DC 1501 Fluid | 40g |
| DC 245 Fluid | 40g |
| Nacre prestige bronze Eckart | 10g |
| Composition **A** | 10g |

0.5g de la composition **B** est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 5. Monomère éthylhexylcyanoacrylate

La composition suivante est réalisée :

| | |
|---|---|
| DC 1501 Fluid | 40g |
| DC 245 Fluid | 40g |
| Nacre prestige bronze Eckart | 10g |
| Ethylhexylcyanoacrylate O-60 de Tong Shen | 10g |

0.5g de la composition est appliqué sur une mèche de 1g de cheveux propre et humide. Après 15 minutes de pause, la mèche est séchée au sèche-cheveux pendant 2 minutes.
La mèche est colorée et la coloration obtenue est résistante au shampooing.

### Exemple 6. Monomère Butylcyanoacrylate

La composition suivante **I** est réalisée :

| | |
|---|---|
| Butylcyanoacrylate B-60 de Tong Shen | 90g |
| acide acétique glacial | 10g |

La composition suivante **J** est réalisée :

| | |
|---|---|
| DC 1501 Fluid | 40g |
| DC 245 Fluid | 40g |
| Nacre prestige bronze Eckart | 10g |
| Composition **I** | 10g |

**1.5g** de la composition **J** est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 7. Monomère éthoxyethyl-cyanoacrylate

La composition suivante **K** est réalisée :

| | |
|---|---|
| DC 1501 Fluid | 37.5g |
| DC 245 Fluid | 37.5g |
| Nacre prestige bronze Eckart | 10g |
| acide acétique glacial | 5g |
| Ethoxyethylcyanoacrylate EO 460 de Tong Shen | 10g |

0.5g de la composition **K** est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 8. Mélange de monomères methylheptylcyanoacrylate et ethylhexylcyanoacrylate

La composition suivante **C** est réalisée :

| | |
|---|---|
| méthylheptylcyanoacrylate de Chemence | 97.5g |
| ethylhexylcyanoacrylate O-60 de Tong Shen | 2.5g |

La composition suivante **D** est réalisée :

| | |
|---|---|
| DC 1501 Fluid | 40g |
| DC 245 Fluid | 40g |
| Nacre prestige bronze Eckart | 10g |
| Composition **C** | 10g |

**1.5g** de la composition **D** est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 9. Mélange de monomères methylheptylcyanoacrylate et butylcyanoacrylate

La composition suivante **E** est réalisée :

| | |
|---|---|
| méthylheptylcyanoacrylate de Chemence | 67.5g |
| butylcyanoacrylate B-60 de Tong Shen | 27.5g |
| Acide acétique glacial | 5 |

La composition suivante **F** est réalisée :

| | |
|---|---|
| DC 1501 Fluid | 40g |
| DC 245 Fluid | 40g |
| Nacre prestige bronze Eckart | 10g |
| Composition **E** | 10g |

**1.5g** de la composition **F** est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 10. Nacre mica-oxyde de titane

La composition suivante est réalisée :

| | |
|---|---|
| DC 1501 Fluid | 40g |
| DC 245 Fluid | 40g |
| Nacre mica-oxyde de titane Prestige Gold Eckart | 10g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.
La mèche est colorée et la coloration obtenue est résistante au shampooing.

### Exemple 11. Nacre mica-oxyde de titane-oxyde de fer

La composition suivante est réalisée :

| | |
|---|---|
| DC 1501 Fluid | 40g |
| DC 245 Fluid | 40g |
| Nacre mica-oxyde de titane-oxyde de fer brun Prestige Sun Gold Eckart | 10g |
| méthylheptylcyanoacrylate de Chemence | 10g |

0.5g de la composition est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 12. Nacre mica synthétique-oxyde de fer

La composition suivante est réalisée :

| | |
|---|---|
| DC 1501 Fluid | 40g |
| DC 245 Fluid | 40g |
| Nacre mica synthétique (fluophlogopite)-oxyde de fer brun Sunshine super bronze de Sun Chemical | 10g |
| méthylheptylcyanoacrylate de Chemence | 10g |

0.5g de la composition est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 13. Nacre mica synthétique-oxyde de titane

La composition suivante est réalisée :

| | |
|---|---|
| DC 1501 Fluid | 40g |
| DC 245 Fluid | 40g |
| Nacre mica synthétique (fluophlogopite)-oxyde de titane Sunshine super blue de Sun Chemical | 10g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.
La mèche est colorée et la coloration obtenue est résistante au shampooing.

### Exemple 14. Pigment titanium black

La composition suivante est réalisée :

| | |
|---|---|
| DC 1501 Fluid | 40 g |
| DC 245 Fluid | 39.2g |
| Pigment noir Titanium Black de Biosynthis | 10g |
| Dispersant polyhydroxystearic acid commercialisé sous le nom de Octacare DSP OL 300 par AVECIA | 0.8g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.
La mèche est colorée et la coloration obtenue est résistante au shampooing.

### Exemple 15. Oxyde de fer noir

La composition suivante est réalisée :

| | |
|---|---|
| DC 1501 Fluid | 40 g |
| DC 245 Fluid | 39.2g |
| Oxyde de fer noir Natpure Black LC 9089de Sensient | 10g |
| Dispersant polyhydroxystearic acid commercialisé sous le nom de Octacare DSP OL 300 par AVECIA | 0.8g |
| méthylheptylcyanoacrylate de Chemence | 10g |

0.5g de la composition est appliqué sur une mèche de 1 g de cheveux propre et humide. Après 15 minutes de pause, la mèche est séchée au sèche-cheveux pendant 2 minutes.
La mèche est colorée et la coloration obtenue est résistante au shampooing.

### Exemple 16. Pigment fluorescent

La composition suivante est réalisée :

| | |
|---|---|
| DC 1501 Fluid | 40 g |
| DC 245 Fluid | 39.2g |
| Pigment fluorescent Sunbrite SG2516 Red orange de Sun Chemical | 10g |
| Dispersant polyhydroxystearic acid commercialisé sous le nom de Octacare DSP OL 300 par AVECIA | 0.8g |
| méthylheptylcyanoacrylate de Chemence | 10g |

0.5g de la composition est appliqué sur une mèche de 1g de cheveux propre et humide. Après 15 minutes de pause, la mèche est séchée au sèche-cheveux pendant 2 minutes.
La mèche est colorée et la coloration obtenue est résistante au shampooing.

### Exemple 17. Quantum Dots

La composition suivante **G** est réalisée :

| | |
|---|---|
| DC 1501 Fluid | 40 g |
| DC 245 Fluid | 40g |
| Quantum Dots (Cdse-ZnS, émission 603 nm) commercialisé par la société Evident Technologie | 10g |
| méthylheptylcyanoacrylate de Chemence | 10g |

**1.5g** de la composition **G** est appliqué sur une mèche de 1 g de cheveux propre et humide. Après 15 minutes de pause, la mèche est séchée au sèche-cheveux pendant 2 minutes.
La mèche est colorée en orange et la coloration obtenue est résistante au shampooing.

### Exemple 18. Quantum Dots en prétraitement

La composition suivante **H** est réalisée :

| | |
|---|---|
| Quantum Dots (Cdse-ZnS, émission 603 nm) commercialisé par la société Evident Technologie | 10g |

La composition suivante **I** est réalisée :

| | |
|---|---|
| DC 1501 Fluid | 45 g |
| DC 245 Fluid | 45g |
| méthylheptylcyanoacrylate de Chemence | 10g |

1 g de la composition **H** est appliqué sur une mèche de 1 g de cheveux propre et humide. La mèche est séchée puis réhumidifiée. 1g de la composition **I** est ensuite appliqué. Après 15 minutes de pause, la mèche est séchée au sèche-cheveux pendant 2 minutes.
La mèche est colorée en orange et la coloration obtenue est résistante au shampooing.

## Revendications

1. Procédé de coloration des matières kératiniques dans lequel on applique sur les matières kératiniques une composition de coloration comprenant, dans un milieu de coloration approprié, au moins un pigment et au moins un monomère cyanoacrylate de formule (I) 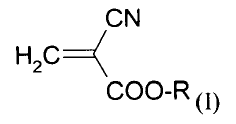 dans laquelle R représente un radical alkyle ou alcoxyalkyle pour la coloration des fibres kératiniques..

2. Procédé selon la revendication 1 dans lequel le ou les monomères cyanoacrylates de formule (I) sont choisis parmi ceux dont le radical R comprend de 1 à 10 atomes de carbone.

3. Procédé selon la revendication 1 ou 2 dans lequel le ou les monomères cyanoacrylates de formule (I) sont choisis parmi ceux comprenant des radicaux R alkyle ou alcoxyalkyle de 6 à 10 atomes de carbones.

4. Procédé selon la revendication 3, dans lequel le ou les monomères cyanoacrylates sont choisis parmi les alkyl(C6-C10)-cyanoacrylates.

5. Procédé selon l'une quelconque des revendications précédentes, dans lequel le monomère cyanoacrylate est un n-octyl 2-cyanoacrylate.

6. Procédé selon l'une quelconque des revendications précédentes dans lequel la quantité de monomères cyanoacrylate est comprise entre 0, 1 % et 80% en poids du poids total de la composition.

7. Procédé selon l'une quelconque des revendications précédentes, dans lequel le ou les pigments sont sous forme de poudre ou de pâte pigmentaire.

8. Procédé selon la revendication 7 dans lequel au moins un pigment est un pigment minéral choisi parmi le dioxyde de titane, traité ou non traité en surface, les oxydes de zirconium ou de cérium, les oxydes de fer ou de chrome, le violet de manganèse, le bleu outremer, l'hydrate de chrome et le bleu ferrique.

9. Procédé selon la revendication 7 dans lequel au moins un pigment est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

10. Procédé selon la revendication 7 dans lequel au moins un pigment est un pigment composite composé de particules comportant un noyau inorganique, au moins un liant assurant la fixation des pigments organiques sur le noyau, et au moins un pigment organique recouvrant au moins partiellement le noyau.

11. Procédé selon la revendication 7 dans lequel au moins un pigment est une laque constituée par un substrat inorganique choisi parmi l'alumine, la silice, le borosilicate de calcium et de sodium ou le borosilicate de calcium et d'aluminium, et l'aluminium sur lequel est adsorbé un colorant.

12. Procédé selon la revendication 7 dans lequel au moins un pigment est un pigment à effets spéciaux choisi parmi les pigments nacrés, les pigments à effets interférentiels non fixés sur un substrat, les pigments fluorescents, les pigments phosphorescents, les pigments photochromiques, les pigments thermochromiques et les quantum dots.

13. Procédé selon la revendication 12, dans lequel le ou les pigments nacrés sont choisis parmi le mica recouvert de titane, ou d'oxychlorure de bismuth, le mica recouvert de titane et d'oxydes de fer, le mica recouvert de titane et de bleu ferrique ou d'oxyde de chrome, le mica recouvert de titane et d'un pigment organique tel que défini à la revendication 15, les pigments nacrés à base d'oxychlorure de bismuth.

14. Procédé selon la revendication 11 dans lequel le ou les pigments à effet interférentiel non fixés sur un substrat sont choisis parmi les cristaux liquides, les paillettes holographiques interférentielles.

15. Procédé selon l'une quelconque des revendications précédentes, dans lequel le ou les pigments sont des pigments colorés.

16. Procédé selon l'une quelconque des revendications précédentes dans lequel le ou les pigments sont chacun présents à des concentrations comprises entre 0,05 et 50 % du poids total de la composition, de préférence de 0.1 à 35 %.

17. Procédé selon la revendication 1 dans lequel la taille du pigment est de 10 nm à 200µm.

18. Procédé selon l'une quelconque des revendications précédentes dans lequel la composition appliquée est anhydre.

19. Procédé selon l'une quelconque des revendications précédentes dans lequel le milieu de coloration est choisi parmi les alcools aromatiques, les alcools gras, les polyols modifiés ou non , les silicones volatiles ou non, les huiles minérales, organiques ou végétales, les cires oxyéthylénées ou non, les paraffines, les alcanes, les acides gras, les amides grasses, les esters gras.

20. Procédé selon l'une quelconque des revendications précédentes comprenant l'application d'un agent nucléophile.

21. Procédé selon la revendication 19 dans lequel l'agent nucléophile est l'eau.

22. Procédé selon l'une quelconque des revendications précédentes dans lequel une première étape qui consiste à appliquer une composition contenant le ou les pigments et une seconde étape qui consiste à appliquer une composition contenant le monomère cyanoacrylate de formule (1), l'agent nucléophile étant présent dans la composition contenant le pigment ou dans une composition séparée.

23. Procédé selon la revendication 22 dans lequel la composition contenant le ou les pigments est une composition aqueuse de pigments et la composition qui contient le ou les monomères cyanoacrylate est anhydre.

24. Procédé selon l'une quelconque des revendications 21 à 23 pour la coloration des fibres kératiniques.

25. Kit de coloration comprenant une première composition qui contient au moins un pigment, une seconde composition qui contient le ou les monomères cyanoacrylates de formule (I) tel que défini aux revendications 1 à 6 et une troisième composition qui contient un agent nucléophile.

26. Kit selon la revendication 25 dans lequel la composition contenant le ou les pigments et le ou les monomères cyanoacrylate sont présents dans la une même composition anhydre.

27. Composition de coloration comprenant, dans un milieu de coloration approprié, au moins un pigment en quantité supérieure à 5 % en poids du poids total de la composition et au moins un monomère cyanoacrylate de formule (I) telle que définie à l'une quelconques des revendications 1 à 6 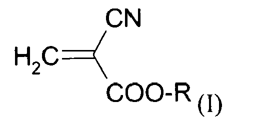 dans laquelle R représente un radical alkyle ou alcoxyalkyle comprenant dans un milieu.
